# EUROPEAN PATENT SPECIFICATION

(11) **EP 0 374 282 B1**
(45) Date of publication and mention of the grant of the patent: **14.09.1994**
(21) Application number: 88121327.6
(22) Date of filing: 20.12.1988
(51) Int. Cl.: C12N 15/53, C12N 9/04, C12N 15/81

(54) **A mutant strain of a methylotrophic organism and a process for the production of a protein in a methylotrophic organism**
Mutanter Stamm von einem methylotrophen Organismus und Verfahren zur Herstellung eines Proteins in methylotrophen Organismus
Souche mutante d'un organisme méthylotrophique et procédé de préparation d'une protéine chez un organisme méthylotrophique

(43) Date of publication of application: 27.06.1990
(73) Proprietor: Rhein Biotech Gesellschaft für biotechnologische Prozesse und Produkte mbH, 40233 Düsseldorf (DE)
(72) Inventor: Roggenkampe, Rainer O, Dr., D-4000 Düsseldorf 13 (DE); Janowicz, Zbigniew A., Dr., D-4006 Erkrath 1 (DE); Hollenberg, Cornelis P., Prof. Dr., D-4000 Düsseldorf 13 (DE)
(74) Representative: Grünecker, Kinkeldey, Stockmair & Schwanhäusser Anwaltssozietät

(56) References cited:
- EP-A- 0 173 378
- EP-A- 0 183 071
- EP-A- 0 242 007
- MOL GEN GENET, vol. 202, 1986, pp. 302-308, Berlin, WG; R. ROGGENKAMP et al.
- CHEMICAL ABSTRACTS, vol. 109, no. 11, 12 Sep. 1988, abstract no. 89504g, Columbus, Ohio, US; L. V. BYSTRYKH et al.
- MOL GEN GENET, vol. 194, 1984, pp. 489-493, Berlin, WG; R. ROGGENKAMP et al.
- BIOTECHNOLOGY, vol. 5, May 1987, pp. 479-485, New York, USA; J. M. CREGG et al.
- BIOSIS DATABASE, abstract no. 87108313; R. ROGGENKAMP et al.
- BIOCHEMICAL SOCIETY TRANSACTIONS, vol. 16, no. 6, 1988, pp. 1081-1083, Cugland; P.E. SUDBERY et al.

## Description

The invention relates to a mutant strain of a methylotrophic organism, a process for the production of a protein in a methylotrophic organism and a method for the selection of methanol oxidase deficient mutants of methylotrophic organisms.

The group of methylotrophic organisms comprises bacteria and yeasts which are able to utilize C₁-compounds as their sole carbon and energy source. The pathways of C₁-metabolism have been investigated for several bacteria as well as for yeast especially for Hansenula polymorpha and Candida boidinii (1,12,13,14,15,16).

In general, the first step in the C₁-metabolism is the oxidation of methanol to formaldehyde and H₂O₂, which reaction is catalysed by the enzyme methanol oxidase (MOX, EC 1.1.3.13). Formaldehyde is dissimilated further to CO₂ by the action of formaldehyde dehydrogenase and formate dehydrogenase. The H₂O₂ formed in these reactions is split into H₂O and O₂ by catalase.

Alternatively, methanol is assimilated into cellular material. After conversion into formaldehyde, it is converted via the xylulose monophosphate pathway into carbohydrates. Dihydroxyacetone synthase (DHAS) plays a crucial role in this assimilation process.

The appearance of MOX, formate dehydrogenase, formaldehyde dehydrogenase, DHAS and catalase is subject to glucose repression, although MOX is derepressed by growing in 0.1% glucose. After induction by growth on methanol, large microbodies, the peroxisomes, are formed. Under these conditions 80% of the volume of the cell consist of peroxisomes, whereas the peroxisomal fraction in glucose-grown cells seems to be very small. The conversion of methanol into formaldehyde and H₂O₂ as well as the degradation of H₂O₂ have been shown to occur in these peroxisomes, which bear some of the key enzymes of methanol utilisation, like MOX catalase and DHAS. This process is a perfect example of compartmentalization of toxic products, of a strong co-ordinate derepression of several cellular processes and of the selective translocation into specific organelles of at least three of the enzymes involved in this process.

Recently the structural gene and the regulatory DNA sequence coding for the methanol oxidase of the yeast Hansenula polymorpha have been isolated (2). According to the nucleotide sequence data obtained the structural gene encodes a protein of 664 amino acids and does not contain any intervening sequence.

Methanol oxidases have several industrial applications. They can for instance be used as bleaching agents in the production of detergents, for the analytical determination of alcohols, and for a elimination of traces of oxygen in food (3,4,5). The problem involved in manufacturing of commercial oxidases is however that oxidases free of contaminating enzymes cannot be produced at low costs. Especially contamination With catalases is decreasing the functional value of oxidase preparations.

Bystrykh et al., (20) disclose mutant strains of Hansenula polymorpha being defective in the synthesis of methanol oxidase, formate dehydrogenase, dihydroxy acetone synthase or dihydroxy acetone kinase. EP-A-0242007 (21) discloses catalase deficient strains of Hansenula polymorpha for use for the production of catalase free oxidase.

Attempts have been made to use the methanol responsive DNA sequences for expression of proteins in Hansenula polymorpha. In EP-A-0374282 (8), a system suitable for expression of protein under the control of the promotor derived from the formate dehydrogenase gene is proposed. In contrast to MOX, DHAS and catalase 1, the two enzymes FMD and FMDH are cytoplasmic proteins. The respective recombinant DNA vector allows expression of up to 25% of the total cell protein under control of the FMDH regulon.

EP-A-173378 (5) discloses the expression of methanol oxidase in auxotrophic mutants of the methylotrophic yeast Hansenula polymorpha. For said purpose, the coding regions of a methanol oxidase are placed under control of the regulatory regions of the MOX gene and were introduced into methylotrophic yeast via episomal vectors or integration into the Hansenula polymorpha genome.

Application of this system for the production of heterologous protein in yeast yields high amounts of protein. However according to the inventor's experience, the expression of some proteins, for example the expression of the homologous MOX protein in the above mentioned system does not yield the expected degree of expression. It is assumed that Hansenula polymorpha has a complex regulation mechanism limiting the amount of some proteins.

Roggenkamp et al., (16) disclose in vitro translation of Hansenula polymorpha mRNA yielding methanol oxidase protein. This reference further discloses the regulation of methanol oxidase by glucose repression and derepression as well as the fact that induction of methanol is controlled at the level of transcription.

Roggenkamp et al., (6) disclose an efficient transformation system for Hansenula polymorpha including complementation of isolated uracil auxotrophs by the URA3 gene of Saccharomyces cervisiae. Furthermore, this reference discloses the Hansenula polymorpha derived ARS sequence HARS1.

Cregg et al. (15) report about the expression of an heterologous protein in another methylotrophic yeast, Pichia pastoris, the expression being controlled by a methanol inducible promotor derived from one of the alcohol oxidase genes of Pichia pastoris. The authors constructed a plasmid containing an expression cassette comprising the HBsAg gene sequence flanked by a fragment carrying the alcohol oxidase 1 (AOX1) promotor fragment and another fragment containing sequences 3' of the AOX1 gene. The expression cassette was introduced into the Pichia pastoris chromosome using a modified version of the 1-step gene replacement method. Upon induction of a thus modified Pichia pastoris strain small amounts of alcohol oxidase due to expression of the AOX2 gene and expression of HBsAg in an amount of 2 to 3% of the soluble protein was observed. For industrial purposes, however, it is desirable to provide an even higher expression rate than observed with the Pichia system.

It is an object of the present invention to provide an improved system for the production of protein in methylotrophic organisms.

Subject of the present invention is a mutant strain of a methylotrophic yeast, wherein the original chromosome of said methylotrophic yeast is defective in effecting synthesis of active methanol oxidase and of active catalase, said mutant having obtained at least one expression cassette comprising a 5' transcriptional regulon of a methanol inducible gene, a DNA sequence coding for a protein to be expressed and optionally a terminator, wherein expression of said DNA sequence is under control of said regulon.

A further subject of the present invention is a process for the production of a protein in a methylotrophic organism wherein a mutant of the present invention is cultivated and protein synthesis is induced.

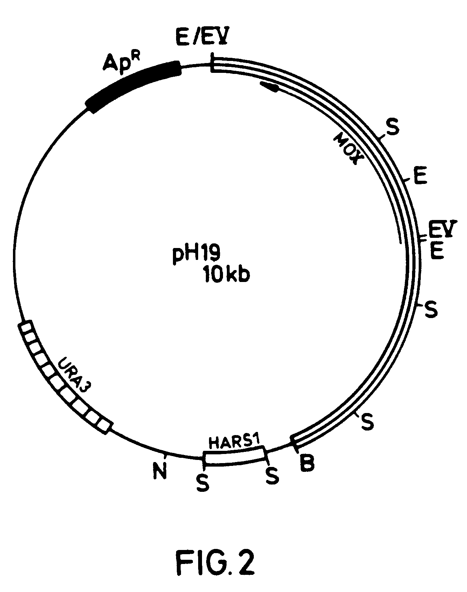

The invention is now described in a more detailed manner by the following description, examples and figures.
The figures show :

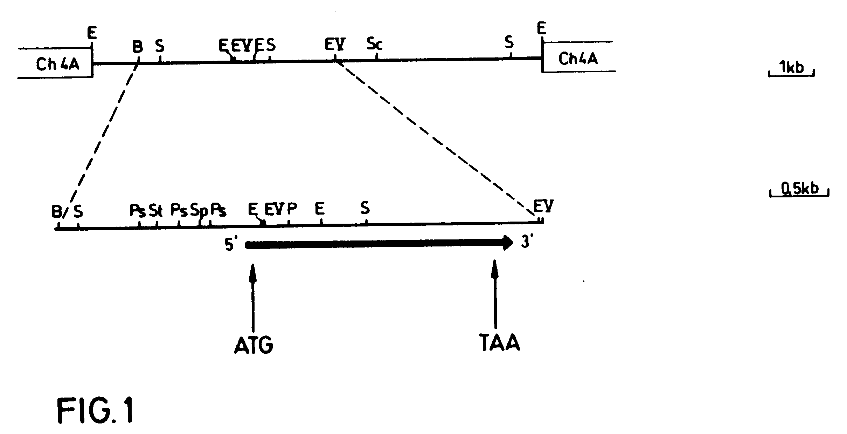

- Fig.1 :: The restriction map of relevant parts of clone lambda charon 4A bearing the MOX gene of Hansenula polymorpha. The construction of this clone is outlined in example I. The clone is also described in (2,5 and 7). The following abbreviations are used :
B : BamHI
E : EcoRI
EV : EcoRV
P : PvuI
Ps : PstI
S : SalI
Sc : SacI
Sp : SphI
St : StuI
- Fig. 2: Plasmid carrying the MOX regulon and the MOX structural gene: pH19;
HARS1: Hansenula polymorpha autonomously replication sequence 1;
Ap^{r} : beta-lactamase gene encoding ampicillin resistance;
URA3: orotidine 5' phosphate decarboxylase gene from Saccharomyces cerevisiae.
The abbreviations used for restriction endonucleases are the same as in Fig. 1.

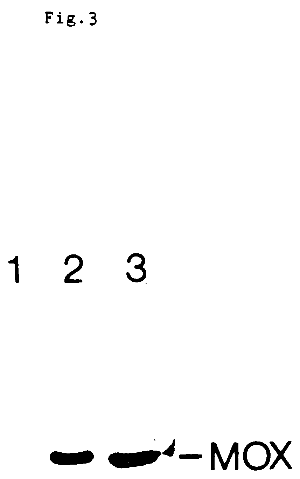

- Fig.3: Immunoblot of MOX protein in crude extracts of Hansenula polymorpha mutants 1N and 3N. Cells were grown in YNB supplemented with 3% glycerol and 1% methanol. An aliquot of 30 ug of protein was applied to each slot. Electrophoresis was performed on 10% polyacrylamide gels in the presence of SDS: Staining of immuno-signals was as described in Materials and Methods. Lane 1, mutant 3N lacking MOX protein; Lane 2, mutant 1N accumulating inactive MOX protein; Lane 3 strain MCT-75 as a control.

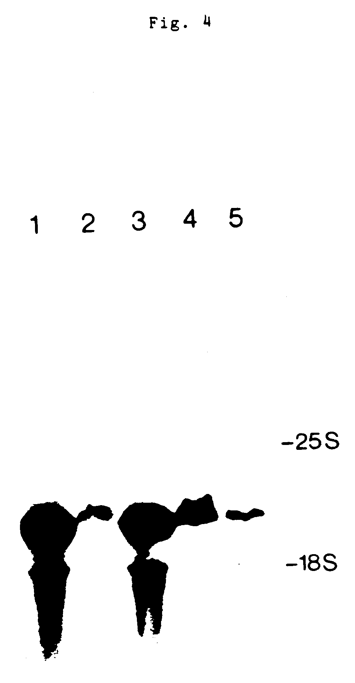

- Fig.4: Northern analysis of MOX-specific RNA in mutants and high-copy number transformants of H. polymorpha. Cells were grown under derepressed conditions with 3% glycerol as a carbon source as described in Table 1. Each sample contained 30 ug of total RNA. MOX-specific RNA, was probed with nick-translated plasmid pH19 using α³² P-dATP and visualized by autoradiography. Lane 1, overproducing transformant Q3N; lane 2, mutant 3N; lane 3, overproducing transformant Q1N; lane 4, control strain MCT-75; lane 5, strain 1N. Positions of the 18 S and 25 S RNA-species corresponding approximately to 1.8 and 3.3 kilobases, respectively are indicated.

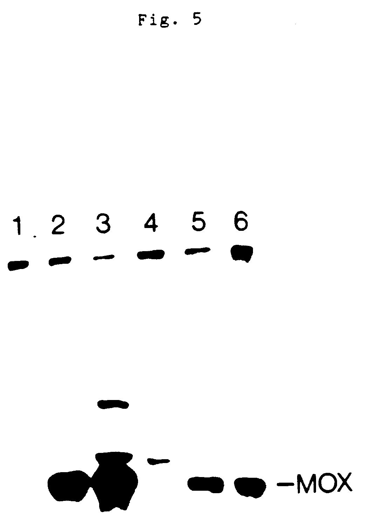

- Fig.5: Cell-free translation in a rabbit reticulocyte lysate programmed with RNA from H. polymorpha mutants and from high-copy number transformants. The reaction mixtures each contained 10 ug of total RNA (see Fig.4) except that of lane 4 containing 50 ug of total RNA. Aliquots were electrophoresed using 8% SDS gels and subjected to fluorography as described in Materials and Methods. Lane 1, mutant 3N; lane 2, overproducing transformant Q1N; lane 3, overproducing transformant Q3N; lane 4, as lane 1, but 50 ug of total RNA used; lane 5, mutant 1N; lane 6, strain MCT-75 (control).

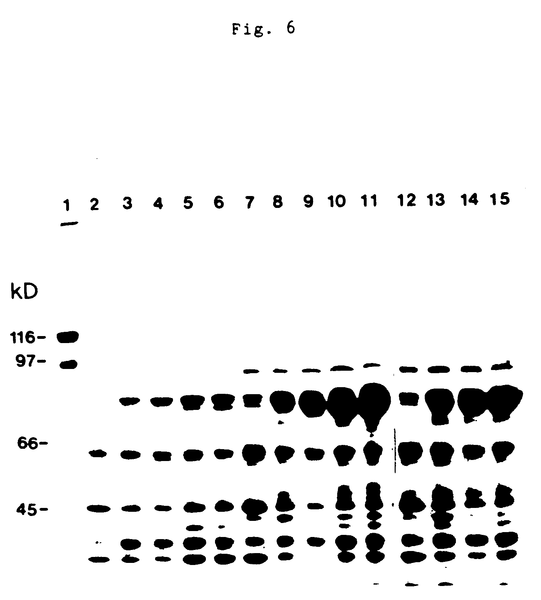

- Fig. 6: Polyacrylamide gel electrophoresis of crude extracts from H. polymorpha cells transformed with the MOX gene on a high-copy number plasmid. Yeast cells were cultivated as described in the legend of Fig. 3. Lane 1: molecular weight markers from top to bottom: β-galactosidase, phosphorylase B, bovine albumin, egg albumin. Lanes 2 - 6: strain MCT-75 grown for 0, 8, 16, 27, 42 h, respectively. Lanes 7 - 11: overproducing transformant Q1N; lanes 12 - 15: overproducing transformant Q3N. Incubation times are identical to those for lanes 2 - 6. The incubation time of 45 h for transformant Q3N is not shown.

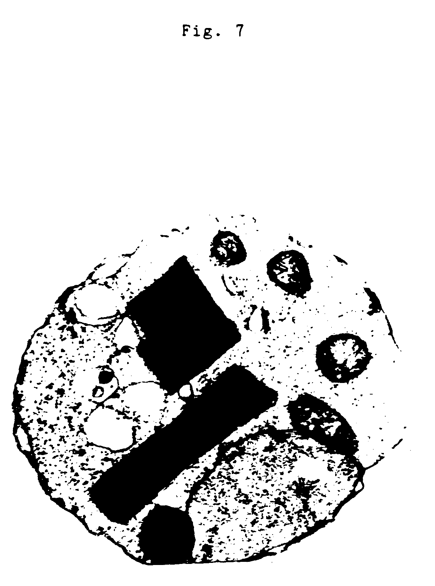

- Fig. 7: Electron micrograph of peroxisomes isolated from H. polymorpha transformants overproducing methanol oxidase.

Throughout this application various publications are referenced by numbers within parentheses. Full citations for these refences may be found at the end of the specification as an annex, listed according to their number immediately following the specification.

The mutant strain according to the present invention may be any mutant of a methylotrophic yeast which is defective in effecting synthesis of active methanol oxidase and active catalase. The mutation may affect any event involved in expression of the structural gene in question, i.e., it may be for example on the regulatory level; it is also possible that the regulation of the expression is not impaired whereas the activity of the synthesized molecule is. The present invention provides examples for both mentioned possibilities. Moreover, since regulatory mutations often exert pleiotropic effects on the affected organism the present invention is not limited to mutants lacking activity of the above specified methanol inducible proteins, but is also directed to mutations of further genes which are involved in methanol metabolism. The original copy of the structural gene coding for the proteins in question may contain small mutations, for example small deletions, transitions or transversions but preferably it is not deleted nor replaced by any other gene.

Some of the enzymes involved in methanol metabolism of yeast have been purified and characterized (17,18). Especially methanol oxidase (EC 1.1.3.13) has been studied in detail. It is an octamer consisting of identical momomers with an apparent molecular weight of about 74 kD and it contains FAD as a prosthetic group. Under derepressed conditions up to 20% of the cellular protein consists of methanol oxidase.

According to the present invention, the activity of the MOX protein is completely abolished in the respective mutant strain. As discussed above lack of activity may be caused by mutations affecting genes involved in the regulation of expression or may be due to synthesis of an inactive protein which however is still detectable for instance by use of immunoassays.

Mutant strains according to the present invention are further free of active catalase; this is of great importance for the isolation of proteins either from the organelles mentioned above, the so called peroxisomes where catalase is located, or the cytoplasma of the respective host, without any contamination by catalase. This is important when the expressed proteins are any type of oxidases, since catalase would destroy the oxidized compounds just created. The provision of a catalase deficient methylotrophic organism is thus a considerable step forward to pure proteins obtained by recombinant DNA technology.

The mutant strains according to the present invention are obtained from methylotrophic yeasts. Examples of methylotrophic yeast are of the genera Pichia, Candida and Hansenula; preferred strains are derived from the species Pichia pastoris, Pichia methylotrophica or Candida boidinii and especially Hansenula polymorpha.

The present invention provides mutant strains having the above mentioned properties of being catalase deficient and in addition being defective in providing active methanol oxidase molecules. The strains disclosed are both derived from the yeast Hansenula polymorpha and called Hansenula polymorpha 1N and Hansenula polymorpha 3N. Both these strains have been deposited with the Deutsche Sammlung von Mikroorganismen and Zellkulturen GmbH and received the deposition numbers DSM4888 (Hansenula polymorpha 1N) and DSM4889 (Hansenula polymorpha 3N). Both these strains lack activity of catalase and methanol oxidase. However, the mutations seem to be different. Mutant 1N accumulates inactive MOX protein, mutant 3N does not show cross reacting materials on immunoblots and thus is considered to have no endogenous production of MOX at all (see Figure 3 and Table 1).

Mutant 1N and mutant 3N are derived from Hansenula polymorpha strain MCT-75 (CAT1, URA3) which is a methylotrophic yeast being deficient for synthesis of active catalases. MCT-75 has been deposited with the Deutsche Sammlung von Mikroorganismen und Zellkulturen and received the deposition number DSM4890. Strain MCT-75 was obtained by EMS mutagenesis of the homothallic strain Hansenula polymorpha ATCC 34438. In addition to being catalase deficient strain MCT-75 is deficient for the expression of orotidine 5'-phosphate decarboxylase (URA3).

In order to obtain high-levels of expression of any desired protein, it is necessary, to modify one of the strains described so far by introduction of the gene whose expression is desired under control of a regulon of a methanol inducible gene. This is conveniently done by introduction of at least one, preferably more than one expression casette wherein each of said expression casettes comprises a regulon of a methanol inducible gene or a functionally equivalent sequence, a DNA sequences coding for the protein to be expressed and optionally a terminator wherein the expression of said DNA sequences is under control of said regulon.

The term "regulon" as used in the application comprises any cis-acting DNA-sequence involved in the regulation of the respective structural gene. The expression thus embraces sequences preceding the respective structural gene, for example, a promotor and sequences which are recognized by transcription factors or other proteins. The meaning of the term "regulon" is thus not limited to sequences corresponding to known promoters. Also included are DNA-sequences exhibiting the same responsiveness to methanol as known regulons of methanol inducible genes, i.e. all functionally equivalent sequences.

The DNA sequence may code for any gene. This gene may be of prokaryotic or eukaryotic origin. The requirements for an appropriate design concerning a plasmid for expression in eukaryotic or prokaryotic organisms respectively, are well known to the person skilled in the art.

The terminator may be any terminator known in the art which terminates transcription in the respective host organism. Terminators may be for example sequences prone to formation of hair pin structures and/or polyadenylation sites. In a preferred embodiment the terminator is derived from the same organism and/or from the same gene as the regulon.

The mutant strain thus constructed provides a convenient tool for high expression of any desired protein in a controlled fashion. Whereas repression of a highly active promoter is not possible in cases of constitutive promoters or is at least not completely tight, the methanol inducible regulons for example of Hansenula are reliably repressed in the absence of methanol and/or presence of glucose in an amount exceeding 0.3%. Protein synthesis is induced by depletion of glucose, and, if desired, synthesis is enhanced by addition of methanol to the culture. Thus the precise control of the present system does not require special media or expensive inducers.

The expression casette may be provided on a recombinant plasmid, comprising one or more selective marker genes and one or more replicons, wherein at least one of these replicons is capable in effecting autonomous replication in said methylotrophic organism. Therefore it comprises marker genes enabling the identification of organisms carrying the respective plasmid. There are several marker genes known in the state of the art. The marker gene used in the inventive plasmid is chosen in accordance with the organisms to be transformed. The plasmid furthermore comprises replicons which are capable of effecting replication in one or more host organisms. It is important to provide a replicon for the replication of the plasmid in the respective methylotrophic host yeast which is a necessary feature in order to propagate a methylotrophic strain carrying said plasmid.

A methylotrophic yeast being a lower eukaryotic organism is able to glycosylate similar to typical eukaryotic patterns in most cases. For the purposes of this invention it is preferred to use the yeast Hansenula polymorpha.

The replication of the plasmid in Hansenula polymorpha is preferrably governed by HARS, which is the Hansenula polymorpha ARS corresponding to other yeast AR sequences. The isolation and identification of this sequence is published in (6).

Another feature of the plasmid according to the invention is the presence of one or more replicons enabling the plasmid to autonomously replicate in a prokaryotic organism. In order to simplify amplification of the DNA and for convenience of handling the plasmid it is possible to include two replicons, for example HARS and an E.coli replicon, for example the Col E 1 origin. Preferably the replicons are derived from the prospective host organisms for establishment of a stably inheritable plasmid.

The plasmid as described above can stay in the transformants as a free, autonomously replicating, high copy number plasmid. In the preferred embodiment the plasmid is present in 10 to 300 copies per cell preferably 30 to 100 copies. Since up to about 300 copies of a plasmid are present in the cell, transcription and translation can occur up to 300 times as effective as in the known system. Therefore in a rather short period of time efficient synthesis of protein occurs which in turn allows the cycle of growth, induction plus synthesis of the desired protein, and harvest to be repeated very quickly. The high gene dosage may partially account for the observed over-expression of the protein.

Integration of the plasmids into the chromosome is a known feature which can be enhanced by provision of plasmids like YIp5(6). Plasmids of these category are prone to integration because of lack of an AR sequence. However also in transformants as described above i.e. mutant strains containing plasmids which are able to replicate autonomously it is possible to obtain transformants having copies of the expression casette integrated into the chromosome. In Hansenula polymorpha integration of these plasmids does occur with a low frequency and spontaneously. It yields strains containing several copies of the plasmid integrated into the genome. These resulting integrants show high mitotic stability of the inserted DNA.

In Hansenula polymorpha it is a special feature that insertion into the chromosome is a non-homologous integration. This is in contrast for example to the mutant strains disclosed by Cregg et al. (15) who obtain a Pichia mutant strain by homologous integration of a single copy of their expression casette into the site of the AOX1 gene of Pichia, thereby replacing the original AOX1 gene.

The number of expression casettes introduced into the Hansenula genome can vary and amount to 1 to 300 copies. Preferred mutant strains contain 2 to 300 or 2 to 150 copies integrated into the genome.

In a further embodiment it is possible that copies of the expression casette are provided as well on plasmids as in the genome. The co-existence of expression casettes on both kinds of molecules is for example a normal intermediate within the course of integration.

The regulon obtained from a methanol inducible gene may be any regulon known to respond to methanol induction. In a preferred embodiment the regulon introduced into the recombinant plasmid is derived from a MOX gene, preferably the MOX-gene of a methylotrophic yeast. Recent investigations have shown that the region controlling the expression of the MOX gene in Hansenula polymorpha comprises an extraordinarily long DNA sequence. Therefore in order to clone the whole regulon it is preferred to clone a fragment of approximately 4 kb upstream of the initiation codon of the MOX gene. Cleavage of the Hansenula polymorpha gene with the restriction endonucleases EcoRV and BamHI yields a DNA fragment of approximately 8 kb containing the MOX regulon and the structural gene. The DNA sequence of the structural gene as well as 2.2 kb of sequences of the environment of the MOX gene are published in Ledeboer et al. (2). From the above mentioned fragment the regulon containing the MOX expression controlling sequences can easily be isolated.

The DNA sequence introduced following the regulon may contain any gene involved in methanol utilisation. Preferred examples are the MOX gene and the genes coding for FMD, DHAS and catalase. It is however also possible to introduce open reading frames having the biological activity of one of the above mentioned proteins without being identical with same.

If the inserted DNA sequence is coding for an enzyme involved in methanol metabolism it is preferred to use homologous genes for expression in an appropriate yeast. The most preferred genes are those obtained from Hansenula. Genes of this organism are very well characterized. The DNA sequences of various methanol metabolism associated genes from Hansenula polymorpha are published (2,5,7).

In a preferred embodiment of the present invention the recombinant plasmid has the identifying characteristics of plasmid pH19. This plasmid contains the Hansenula polymorpha AR sequence 1 provided on a SalI fragment derived from pHARS1. Further components are the URA3 gene, an ampicillin resistence gene and the above mentioned 8 kb fragment containing the MOX structural gene and 4 kb of 5′ non-coding sequences. The construction of this plasmid is outlined in example I below.

Furthermore the system is suitable for expression also of other proteins which are not involved in methanol utilisation and which also not necessarily are derived from the methylotrophic organism in which expression is conducted. Preferred examples of genes introduced into the plasmid according to the invention are the gene for Hepatitis B antigens, any other viral antigen, protease inhibitors, especially trypsin, growth factors, colony stimulating factors, blood anticoagulation factors, factors regulating blood coagulation and clot dissolving factors, bacterial toxins and antigens, antigens from parasites, especially from Plasmodium, and enzymes for the food industry.

The mutant strains containing several copies of the expression casette integrated into the chromosome are obtained by transformation of the respective host organism with a suitable plasmid carrying the expression casette, which plasmid tends to integrate by non-homologous recombination into the Hansenula genome. The copy number of integrated plasmids can be enhanced by several subsequent cycles of growth and transformation using a plasmid carrying the expression casette. By this method mutant strains containing 300 or more chromosomal copies can be obtained.

The present invention also provides a process for the production of a protein in a methylotrophic yeast wherein a mutant of a methylotrophic yeast as described above is cultivated, and wherein protein synthesis is induced. According to the present invention the protein, whose production is desired, is encoded on an expression casette. The expression of the DNA sequence coding for said protein is under control of a regulon derived from a methanol inducible protein. This allows induction of the protein synthesis of any desired gene. The protein thus produced can be isolated from the respective cell by any protein isolation procedure known in the art. In order to achieve high-level expression of the desired gene product, it is of advantage, to use a strain having a copy number of 10 or more as described above. If a plasmid is used, this plasmid has to fulfill the minimum requirements, namely it has to possess one or more selective marker genes, one or more replicons, wherein at least one of the replicons is capable of effecting replication in a methylotrophic organism, a regulon of a methanol inducible gene or a functionally equivalent sequence, a DNA sequence coding for the desired protein and optionally a terminator. In the most preferred embodiment the plasmid used in the process according to the present invention is the plasmid pH19, which is shown in Figure 2. This plasmid is composed of the Hansenula polymorpha autonomous replicating sequence 1, a 4kb fragment preceeding the MOX initation codon comprising the MOX regulon, the MOX-structural gene, an ampicillin resistance gene and the gene for orotidine 5'-phosphate decarboxylase URA3 in order to complement the deficiency of the respective mutant for this gene.

Surprisingly, the protein expressed by a mutant according to the present invention containing plasmid pH19 may represent up to 75% of total cellular protein following derepression. In the case of the expression of perixosomal proteins like methanol oxidase the over-produced protein is transported into the peroxisomes, and is efficiently protected against proteolytic degradation. In over-expressing strains obtained according to the present invention the volume of the peroxisomes is strongly increased. This increase is for example demonstrated in figure 7, which shows strain 3N transformed with the plasmid pH19. The transformed strain has been designated Q3N. Storage of the expressed protein in peroxisomes has the invaluable advantage that due to inhibited proteolysis the stability is increased and subsequent isolation of the expressed protein is facilitated. Presumably foreign proteins containing additional translocation signals can also be transported into the peroxisomes.

The process according to the present invention is highly advantageous in that growth of the culture can be carried out using well known standard media, which in general are affordable at low cost. If expression of the cloned gene is desired to start, the culture is allowed to deprive of carbon - sources, which might prevent induction of the system. Upon depletion derepression can occur. Furthermore in strains not being catalase deficient while having an active MOX protein the level of protein synthesis can be further enhanced by addition of low amounts of methanol. Addition of methanol increases the rate of expression about 3 to 10 times.

The process described above results in a overexpression of the protein encoded by the expression casette. As already mentioned above the desired protein may represent up to 75% of the total cellular protein following derepression and/or additional induction by addition of methanol. According to the inventor's knowledge the process of comparable efficiency using eukaryotic organisms has not been known before.

The protein obtained by the process according to the present invention is totally devoid of catalase activity, since the host organisms do not synthesize any active catalase. This enables the isolation and provision of proteins being totally free of catalase activity. In a preferred embodiment a methanol oxidase is obtained.

The methanol oxidases produced according to the present invention are widely applicable in industry. For example this enzyme is used in commercial detergents since the hydrogen peroxide produced acts as a bleaching agent. Furthermore methanol oxidases are used for the detection of methanol and also ethanol. Although in vivo only methanol is metabolized it could be demonstrated in vitro that the enzyme also reacts with ethanol. Both kinds of reactions can be used for determination of the respective alcohol. A further important application is scavenging of oxygen in food. In the presence of oxygen phenolic hydroxyl groups are oxidised thus producing a slight change in colour of the food. Furthermore oxygen enables growth of a variety of microorganisms. In order to avoid these effects methanol oxidases are added to food.

For the provision of methanol oxidase deficient strains of a methylotrophic organism a surprisingly elegant selection system has been found. Since the selection of MOX deficient mutants normally proves to be very difficult the present inventors designed a system which makes use of the methylotrophic properties of the strains themselves. The procedure is based on the fact that methylotrophic organisms being deficient for expression of catalase are unable to grow on a medium containing methanol since, under these conditions expression of the MOX gene is derepressed and methanol oxidase generates toxic hydrogen peroxide. Consequently organisms still producing methanol oxidase will intoxicate themselves upon expression of the MOX gene by accumulation of hydrogen peroxide. Colonies growing on glycerol plates supplemented with 3% methanol are mutants which are not further able to produce hydrogen peroxide. These mutants are then subjected to methanol-oxidase determination assays, thereby identifying MOX-deficient mutants. As determined by the present inventors the spontaneous frequency of such mutations is in the order of 10⁻⁶.

Therefore MOX-deficient mutants of methylotrophic organisms can easily be selected using this method.

The medium used for the selection procedure described above should contain between 1% and 3% glycerol per liter of the medium. The methanol content may vary between 2% and 3% (v/v). In a preferred embodiment YNB-medium (yeast nitrogen base: Difco; 0,67%, pH 5,5) is used for selection of methanol oxidase deficient Hansenula polymorpha from a catalase deficient strain of the same organism.

Mutant strains of a methylotrophic organism containing multiple copies of an expression casette can easily be obtained by growth of Hansenula cells which were transformed with plasmids containing the desired expression casette. Hansenula has the exceptional property of performing non-homologous recombination of the plasmids with its chromosome. For this purpose it is not necessary to provide plasmids having an autonomously replicating sequence. However transformation with a plasmid, being able to replicate inside the cell, has the advantage, that due to the enlarged copy number of the expression casettes available the event of non-homologous recombination is much more likely to occur. The frequency of recombination can further be enhanced by several subsequent cycles of transformation and growth. The method also provides a possibility to insert copies of different genes into the Hansenula genome for example genes of different subunits of a complex enzyme.

In the most preferred embodiment the preparation of mutants strains of a methylotrophic organism containing multiple copies of the expression casette is performed by transforming Hansenula with a autonomously replicating plasmid, preferably a plasmid of high copy number. Upon transformation the cells are grown on non-selective medium for several generations. Following this incubation period the cells are plated on a selective medium. Due to the loss of plasmids during the growth period on non-selective medium the following incubation on selective medium selects for organisms containing integrated copies of the selective marker and thus also for the presence of at least one expression casette. These integrated copies have been shown to be mitotically stable.

In a preferred embodiment the selection procedure is performed using strain MCT-75, which is deficient for production of active catalase, for plating on a methanol containing medium. As outlined above, MCT-75 is not able to grow on methanol containing plates if the MOX regulon is derepressed and thus hydrogen peroxide is created inside the cell. MCT-75 has the advantages of being a very well characterised strain which also lacks the function of the orotidine 5′-phosphate decarboxylase and thus is convenient for cloning procedures using the common selection marker URA3.

As already mentioned above a plasmid having the identifying characteristics of pH19 is the preferred plasmid for the performance of the processes according to the present invention and also for the method for the preparation of mutant strains containing multiple copies of an expression casette.

### Materials and Methods

### I. Materials :

1. Strains
   a) a thermophilic homothallic strain of Hansenula polymorpha (ATCC 34438) was used as a wild type strain
   b) mutant strain MCT-75 was obtained by treating a wild type strain with EMS according to known procedures. All mutants obtained were selected for their unability to grown on methanol (met mutants). Subsequently the activity of enzymes involved in methanol metabolism was determined. Mutant MCT-75 (CAT1, URA3) did not show any catalase activity. For a detailed description see Example I.2.
   c) Mutants 1N and 3N are mutants of MCT-75 selected as described below. (Example I.3)
2. Media:
   a) Yeast was grown at 37°C on minimal YNB medium as described (6,7). Carbon sources were supplemented as indicated. If necessary 20 ug/ml medium uracil were added.
   b) Media used for growth of Escherichia coli are described previously (9).
3. Vectors:
   a) Lambda vector Charon 4A is described in (9).
   b) Yeast vector pHARS 1 is published in (6).

### II. Methods:

1. A gene library of Hansenula polymorpha DNA was constructed as described in (2,7).
2. RNA from Hansenula polymorpha was isolated as described in (7). Analysis by in vitro translation of mRNA was conducted in a rabbit reticulocyte lysate obtained from Amersham. The reaction mixture consisted of 8 ul lysate, 1 ul ³⁵S methionin (15uCi/ml, purchased from Amersham)and 10 ug (corresponding to 2 ul) of total RNA.
3. Northern blot analysis was performed essentially as described by Maniatis et al. (9). RNA was resolved using formaldehyde gels and blotted onto nitrocellulose.
4. Analysis of in vitro translation products was performed by subjecting aliquots of 6 ul of the reaction mixture to SDS polyacrylamide electrophoresis (10). Protein bands were visualised by fluorography with sodium salicylate.
5. Transformation of Hansenula polymorpha was effected using the protoplast method essentially as described in (6).
6. Electron microscopy : Cells were converted to protoplasts using Zymolyase (Miles) as described for transformation of yeast (6) and washed with 1.2 M sorbitol in 0.05 M cacodylate buffer, pH 7.0. Fixation was for two hours in the same solution containing 4% glutaraldehyde. The samples were post-osmicated for 18 hours. After repeated washings of decreasing sorbitol concentrations the cells were embedded in 0.75% low melting agarose in cacodylate buffer. Agar cubes were cut, dehydrated in a graded ethanol series and finally embedded in Spurr Epon. Thin sections were carried out on an LKB Ultrotome III and poststained with uranyl acetate (60 minutes) followed by lead citrate (30 minutes). Sections were examined in a Philips EM 300 electron microscope.
7. Determination of the enzymatic activity of methanol oxidase was performed by measuring the oxidation of ABTS (2.2'-Azino-di-(3-ethyl-benzthiazolinsulfonate Diammoniumsalt (C₁₈H₁₆N₄O₆S₄-(NH₄)₂; M:548,7) (Boehringer). Methanol oxidase activity is determined by combining 750 ul H₂O, 200 ul 0.1M K-PO₄-buffer pH 7.5 and 10 ul of a solution containing ABTS (50 mg/ml) with 5 ul sample, 10 ul substrate (methanol) and 20 ul of a peroxidase and incubating the reaction mixture at 30°C. Upon generation of H₂O₂ by action of oxidase one mole H₂O₂ serves to oxidise two moles of ABTS. The concomitant increase of absorption observed at a wavelength of 420 nm corresponds to the MOX-activity contained in the sample.
8. Specific antibodies against methanol oxidase, catalase, and dihydroxyaceton synthase were obtained by immunization of rabbits using standard methods.
9. Immunoblots (11) of proteins on nitrocellulose after separation in SDS polyacrylamide gels were stained with protein A-conjugated peroxidase (Amersham) and 3,3′-diaminobenzidine.

### Example I:

### Construction of strains showing MOX over-expression.

### 1. Construction of pH19:

A gene library of Hansenula polymorpha DNA was constructed in Lambda Charon 4A as described earlier (2,7). Charon 4A clones carrying the MOX gene were isolated as described in (2) and (5).

5 microgram DNA of clone L47 carrying the MOX gene were cleaved with restriction endonuclease BamHI and subsequently subjected to a partial digestion using EcoRV. The resulting fragments were resolved on a 1% agarose gel according to conventional procedures and the desired fragment of 4.0 kb size recovered by electroelution.

Plasmid pHARS 1 (6) was isolated according to known procedures. 2 microgram of isolated DNA were cleaved using restriction endonuclease EcoRI and subjected to a treatment with Klenow polymerase in presence of dNTP. The blunt-ended linear molecules were then cleaved with BamHI and purified.

The DNA fragment encompassing the MOX gene and its regulon (EcoRV-BamHI fragment) was ligated with the pHARS vector prepared as described above. The ligation mixture was used to transform E.coli cells. E.coli transformants were screened for the desired plasmids by submitting same to restriction analysis. Plasmid pH19 showing the expected restriction pattern was chosen for further experiments. A map of this plasmid is shown in figure 2.

### 2. Construction of MCT-75

Mutant strain MCT-75 was obtained by mutagenesis using the conventional mutagen EMS (ethylmethylsulfonic acid). A 10 ml culture of wildtyp Hansenula polymorpha was grown to an optical density of about A₆₀₀ = 3.0, harvested by centrifugation, washed twice using 0.1 M sodium phosphate buffer and resuspended using the same buffer. EMS was added up to a final concentration of 3% v/v and the mixture was incubated for 3 hours at 37°C. Following this incubation period the cells were subjected to subsequent resuspension and centrifugation steps using phosphate buffer, 6% sodium thiosulfate and two cycles of phosphate buffer. The cells were then incubated 2 days in YEPD medium (1% Bacto Pepton, 2% yeast extract (Difco), 2% glucose, pH6) at 30°C and subsequently plated onto rich media (YEPD); growing colonies were then checked for their unability to grow on methanol as the sole carbon source by replica-plating on YNB-methanol selective medium. The mutants thus obtained (met-mutants) were tested for their activity of the various enzymes involved in methanol utilization, i.e. MOX-protein, DHAS, FMDH, catalase etc. One mutant was found to have lost catalase-activity. Presence of inactive catalase-molecules could be detected using appropriate immunoassays. This mutant was subjected to the procedure described in (6) in order to introduce a mutation in the URA3 gene.

### 3. Construction of MOX-deficient mutants of MCT-75

MOX-deficient mutants of strain MCT-75 were obtained by plating MCT-75 on agar-plates containing glycerol and 3% methanol as carbon sources. Under these conditions catalase-deficient strain MCT-75 does not grow (see above). Growing colonies occured with a frequency of about 10⁻⁶.
The mutants obtained were tested for MOX activity using the enzymatic assay and furthermore analysed via an immunoassay. Two mutants were identified not to exhibit any MOX-activity (see Table 1). Mutant 1N was shown to accumulate inactive MOX-protein in addition to inactive catalase, whereas mutant 3N did not produce detectable amounts of MOX-protein at all (Fig. 3).

### 4. Construction of Q1N and Q3N

Mutant strain 1N and 3N were transformed with plasmid pH19 be selecting uracil prototroph colonies. For transformation the protoplast method as described in (6) was used. The resulting strains were named Q1N and Q3N, resp.

### Example II: Characterization of Hansenula polymorpha mutants

### 1. Northern analysis

The RNAs of strains MCT-75, 1N, 3N, Q1N and Q3N were subjected to electrophoresis using formaldehyde gels and subsequently blotted to nitrocellulose. Hybridization was carried out according to conventional procedures (9) using nick-translated pH19 as a MOX specific probe. The resulting autoradiography is shown in Fig. 4. This figure visualises MOX specific mRNA of strains Q3N (lane1), 3N (lane 2), Q1N (lane 3), MCT-75 (lane 4) and 1N (lane 5). It is obvious from this figure that the level of MOX specific mRNA is dramatically increased in both the Q-strains compared to strain MCT-75, ie. the strains containing the plasmid according to the present invention show increased rates of transcription. Furthermore it is obvious, that mutant strains 3N and 1N also produce transcripts.

### 2. In vitro translation

RNA preparations of mutant strains 1N and 3N, transformed strains Q1N and Q3N and strain MCT-75 were used to obtain translation products in a rabbit reticulocyte lysate. The samples were subjected to electrophoresis and visualized by fluorography. The resulting autoradiography shown in Fig. 5 indicates that there is no translation of MOX specific RNA in mutant 3N (lane 1). However, upon using a five fold amount of RNA synthesis of a protein having the size of MOX could also be demonstrated (lane 4). Lanes 2 and 3 show translation products obtained by programming the system with RNA isolated from strains Q1N and Q3N, respectively.

The amount of protein expressed does clearly far exceed the amount observed for strain MCT-75 (lane 6) and mutants 1N and 3N.

Two additional protein bands of higher molecular weight than MOX appear in strain Q1N that are apparently induced by MOX over production (Fig.5,lane 3). The nature of these proteins is still unknown.

### 3. Expression of MOX Protein in vivo

In order to estimate the quantity of MOX activity and protein synthesised in vivo crude extracts of yeast cells grown on glycerol were analyzed. Due to the inactivity of their catalase these strains cannot grow in media containing methanol as the only carbon source.

The specific activities of the respective MOX proteins observed for the different strains are summarized in Table 1. As is obvious from this Table strains 1N and 3N do not exhibit any MOX activity. The specific activity of MOX protein in strains Q1N and Q3N was about ten times higher than in strain MCT-75. Since purified MOX protein has a specific activity of 5.5 U/mg protein the transformants are producing the enzyme in the range of about two thirds of the total cellular protein. This was confirmed by electrophoresis of crude extracts in polyacrylamide gels showing MOX as the predominant band (Fig.6). The quantity of MOX in transformed and non transformed cells is dependent on the growth phase. The highest amounts are observed during the stationary phase (Fig.6, lane 6, 11 and 15). Growth rates are not reduced despite the abundant production of MOX. Since the protein contains FAD as a prosthetic group there is obviously no limitation of this compound in transformed cells in view of the high specific activity. The over-expression of MOX had no effects on the synthesis of other peroxisomal enzymes; the levels of DHAS and FMDH were shown to be normal in immunoassays.

Since in Hansenula polymorpha expression of MOX is strictly regulated by glucose repression (16) the transformants could possibly by-pass this regulation by titrating out a regulatory factor. However in transformants grown on glucose also no MOX activity could be detected (Table 1). Thus glucose repression of MOX is independent of the copy number of the gene.

The above example shows a surprisingly efficient system for production of active MOX protein in a strain lacking any catalase activity.

### 4. Electron Microscopy of Peroxisomes

Peroxisomes in MOX-transformed strains show a drastic increase in size and a different morphology im comparison to control cells (Fig. 7). They rather exhibit rectangular instead of round or elliptic forms and the crystalloid core can completely fill up the original lumen (Fig. 7). These structural changes are an obvious explanation of the frequent failure to isolate intact organelles by cell fractionation.

Thus, EM data confirm the suggestion that most of the synthesized MOX protein is present in peroxisomes. About 75% of total cell protein of the transformants is active MOX.

**Table 1**

| Activities of methanol oxidase in mutants and high-copy number transformants of Hansenula polymorpha | | |
|---|---|---|
| Strain | Specific activity * | |
| | Carbon source | |
| | Glycerol | Glucose |
| Mutant 1N | 0.0 | 0.0 |
| Mutant 3N | 0.0 | 0.0 |
| Transformant Q1N | 3.67 | 0.0 |
| Transformant Q3N | 3.71 | 0.0 |
| MCT-75 | 0.32 | 0.0 |

| | | |
|---|---|---|
| *: Specific activity : Micromoles H₂O₂ produced per minute per milligram of protein | | |

Cells were grown until early stationary phase in YNB + 3% of carbon source, respectively. Except for growth of transformants 20 ug of uracil per ml was added to the growth medium;

### References

1. Y. Tani in: Methylotrophs; Hon, C.T., ed.pp 55-85, CRC Press, Bocaraton, USA, 1984.
2. A.M. Ledeboer, L. Edens, J. Maat, C. Visser, J.W. Bos, C.T. Verrips, Z. Janowicz, M. Eckart, R. Roggenkamp & C. Hollenberg, Nucleic Acid Research 13, 3063-3082, 1985
3. J.R. Woodward, Microbiol. Sciences 3, 9-11, 1986
4. BP 2101167 (Unilever)
5. EP-A-0173378 (Unilever)
6. R. Roggenkamp, H. Hansen, M. Eckart, Z.A. Janowicz & C.P. Hollenberg, Mol. Gen. Genet. 202, 302-308, 1986
7. Z.A. Janowicz, M.R. Eckart, C. Drewke, R.O. Roggenkamp, C.P. Hollenberg, J. Maat, A.M. Ledeboer, C. Visser & C.T. Verrips, Nucleic Acid Research 13, 3043-3062, 1985
8. EP-A-0374282 (Rhein Biotech)
9. T. Maniatis, E.F. Fritsch & J. Sambrook, Molecular Cloning, Cold Spring Harbor Laboratory, 1982
10.V.K. Laemmli, Nature 227, 680-685, 1970
11.H. Towbin, T. Staehelin and J. Gordon, Proc. Natl. Acad. Sci. USA 76, 4350-4354, 1979
12.R. Roggenkamp, H. Sahm and F. Wagner. FEBS Lett. 41, 283-286, 1974
13.M. Veenhuis and W. Harder in: Peroxisomes in biology and medicine; H.D. Fahimi and H. Sies (ed.),pp 437-460, Springer Verlag, Berlin, Heidelberg, FRG, 1987
14.M. Veenhuis, J.P. van Dijken & W. Harder, Adv. Microb. Physiol. 24, 1-82, 1983
15.J.M. Cregg, J.F. Tschopp, C. Stillman, A. Siegel, M. Akong, W.S. Craig, R.G. Buckholz, K.R. Madden, P.A. Kellaris, G.R. Davis, B.L. Smiley, J. Cruze, R. Torregrossa, G. Velicelebi & G.P. Thill, Biotechnology 5, 479-485, 1987
16.R. Roggenkamp, Z. Janowicz, B. Stanikowski & C.P. Hollenberg, Mol. Gen. Genetics 194, 489-493, 1984
17.H Sahm in: Advances in Microbiol. Engineering; T.K. Ghose, A. Fiechter and N. Blakebrough (eds), Vol. 6, pp 77-103, Springer-Verlag, Berlin, 1977
18.L.V. Bystryhk, A.P. Sokolov & Y.A. Trotsenko, FEBS Letters 132, 324-328, 1981
19.M. Roa and G. Blobel, Proc.Natl.Acad.Sci. USA 80, 6872-6876, 1983
20. Bystrykh, L.V. et al., Chemical Abstracts 109 (11) No. 89504g, 12.09.1998.
21. EP-A-0242007, Unilever N.V., 21.10.1987.

## Claims (Claims for the following Contracting State(s): AT, BE, CH, LI, DE, FR, GB, GR, IT, LU, NL, SE)

1. A mutant strain of a methylotrophic yeast, wherein the original chromosome of said methylotrophic yeast is defective in effecting synthesis of active methanol oxidase and of active catalase, said mutant having obtained at least one expression cassette comprising a 5' transcriptional regulon of a methanol inducible gene, a DNA sequence coding for a protein to be expressed and optionally a terminator, wherein expression of said DNA sequence is under control of said regulon.

2. The mutant according to claim 1, **characterized in that** the methylotrophic yeast is of one of the genera Pichia, Candida or Hansenula, preferably of the species Hansenula polymorpha, Pichia pastoris, Pichia methylotropica or Candida boidinii.

3. The mutant according to any of claims 1 or 2, **characterized in that** the expression cassette is provided on a recombinant plasmid comprising one or more selective marker genes and one or more replicons, wherein at least one of these replicons is capable of effecting autonomous replication in said methylotrophic yeast.

4. The mutant according to claim 3, **characterized in that** the replicon provided is HARS 1 from Hansenula, or a functionally equivalent sequence.

5. The mutant according to claim 3 or 4, **characterized in that** at least one of the replicons provided on the plasmid is recognized by a prokaryotic host organism.

6. The mutant according to any of claims 3 to 5, **characterized in that** the plasmid is present in a copy number of about 10 to about 300 copies, preferably 30 to 100 copies.

7. The mutant according to claim 1 or 2**, characterized in that** the at least one expression cassette is provided in at least one site of the chromosome.

8. The mutant according to claim 7, **characterized in that** it is obtainable by integration of at least one plasmid carrying the expression cassette into the chromosome of a yeast of the genus Hansenula.

9. The mutant according to claim 7 or 8**, characterized in that** the number of expression cassettes introduced into the genome of said methylotrophic yeast is 1 to about 300, preferably 2 to 300 or 2 to 150.

10. The mutant according to any of claims 1 to 9, **characterized in that** the regulon is derived from the MOX gene, preferably the MOX gene of Hansenula polymorpha.

11. The mutant according to any of claims 1 to 10, **characterized in that** the DNA sequence is coding for a protein having the biological activity of the MOX(methanoloxidase)-protein, FMDH(formate dehydrogenase)-protein or DHAS(dihydroxyacetonesynthase)-protein of a methylotrophic organism.

12. The mutant according to any of claims 1 to 11, **characterized in that** the DNA sequence coding for the protein to be expressed is obtained from yeast, preferably Hansenula polymorpha.

13. The mutant according to any of claims 1 to 12, characterized in that the expression cassette is carried by plasmid pH19 of figure 2, said plasmid being obtainable by combining pHARS1, digested with EcoRI and BamHI, with a 4.0 kb EcoRV-BamHI fragment from the Hansenula polymorpha genome carrying the MOX gene of Hansenula polymorpha in a manner known per se.

14. The mutant according to any of claims 1 to 13, **characterized in that** the DNA-sequence to be expressed is coding for any one of the proteins of the following group:
Hepatitis B antigens, any other viral antigen, protease inhibitors especially trypsin inhibitors, growth factors, colony stimulating factors, blood anticoagulation factors, factors regulating blood coagulation and clot dissolving factors, bacterial toxins and antigens, antigens from parasites, especially from Plasmodium, enzymes for the food industry.

15. A process for the production of a protein in a methylotropic organism **characterized in that** any of the mutants of claims 1 to 14 is cultivated and protein synthesis is induced.

16. A process according to claim 15 **characterized in that** protein synthesis is induced by depletion of repressing carbon sources, and/or optionally by addition of methanol.

17. A process according to claim 15 or 16, **characterized in that** protein synthesis results in over expression of the protein encoded on the expression cassette.

18. A process according to any of claims 15 to 17, **characterized in that** the protein is methanol oxidase.

19. A method for the selection of methanol oxidase deficient mutants of methylotrophic yeast wherein a catalase deficient yeast is grown by using a medium containing methanol and a carbon source in an amount which does not cause repression of methanol inducible genes, and wherein the clones capable to grow on that medium are isolated as methanol oxidase deficient yeasts.

20. A method according to claim 19, **characterized in that** the catalase deficient yeast is a yeast of one of the genera Pichia, Candida or Hansenula.

21. A method according to claim 19, **characterized in that** the catalase deficient yeast is Hansenula polymorpha MCT-75(DSM 4890).

22. A method for the preparation of a mutant strain of a methylotropic yeast containing at least one copy of an expression cassette, the original chromosome of said methylotrophic yeast being defective in effecting synthesis of active methanol oxidase and active catalase, wherein said mutant strain of a methylotrophic yeast is produced by transforming said methylotrophic yeast with a plasmid carrying the expression cassette, optionally followed by integration of the plasmid.

23. A method according to claim 22, **characterized in that** the plasmid is an autonomously replicating plasmid, preferably an autonomously replicating plasmid of high copy number.

24. A method according to claim 23, **characterized in that** the organism after transformation is grown under non-selective conditions for several generations and subsequently mitotically stable transformants containing at least one copy of the expression cassette are selected by growth under selective conditions.

25. A method according to any of claims 22 to 24, **characterized in that** the methylotropic yeast is Hansenula polymorpha 1N or Hansenula polymorpha 3N.

26. Hansenula polymorpha 1N (DSM 4888).

27. Hansenula polymorpha 3N (DSM 4889).

## Claims (Claims for the following Contracting State(s): ES)

1. A method for preparation of a mutant strain of a methylotrophic yeast containing at least one copy of an expression cassette, wherein the original chromosome of said methylotrophic yeast is defective in effecting synthesis of active methanol oxidase and active catalase, and wherein said mutant strain of a methylotrophic yeast is produced by transforming said methylotrophic yeast with a plasmid carrying the expression cassette, said transformation being optionally followed by integration of the plasmid, wherein said expression cassette comprises a 5' transcriptional regulon of a methanol inducible gene, a DNA sequence coding for a protein to be expressed and optionally a terminator and wherein expression of said DNA sequence is under control of said regulon.

2. A method according to claim 1, characterised in that the plasmid is an autonomously replicating plasmid, preferably an autonomously replicating plasmid of high copy number.

3. A method according to claim 2, characterised in that the organism after transformation is grown under non-selective conditions for several generations and subsequently mitotically stable transformants containing at least one copy of the expression cassette are selected by growth under selective conditions.

4. A method according to any of claims 1 to 3, characterised in that the methylotrophic yeast is one of the genera Pichia, Candida or Hansenula, preferably of the species Hansenula polymorpha, Pichia pastoris, Pichia methylotropica or Candida boidinii.

5. The method according to claim 4, characterised in that the methylotrophic yeast is Hansenula polymorpha 1N or Hansenula polymorpha 3N.

6. The method according to any of claims 1 to 5, characterised in that the expression cassette is provided on a recombinant plasmid comprising one or more selective marker genes and one or more replicons, wherein at least one of these replicons is capable of effecting autonomous replication in said methylotrophic yeast.

7. The method according to claim 6, characterised in that the replicon provided in HARS 1 from Hansenula, or a functionally equivalent sequence.

8. The method according to claims 1 to 7, characterised in that at least one of the replicons provided on the plasmid is recognized by a prokaryotic host organism.

9. The method according to any of claims 6 to 8, characterized in that the plasmid is present in a copy number of about 10 to about 300 copies, preferably 30 to 100 copies.

10. The method according to any of claims 1 to 5, characterized in that the at least one expression cassette is provided in at least one site of the chromosome.

11. The method according to claim 10, characterised in that at least one plasmid carrying the expression cassette is integrated into the chromosome of the yeast of the genus Hansenula.

12. The method according to claim 11, characterised in that the number of expression cassettes introduced into the genome of said methylotrophic yeast is 1 to about 300, preferably 2 to 300 or 2 to 150.

13. The method according to any of claims 1 to 12, characterised in that the regulon is derived from the MOX gene, preferably the MOX gene of Hansenula polymorpha.

14. The method according to any of claims 1 to 13, characterised in that the DNA sequence is coding for a protein having the biological activity of the MOX (methanoloxidase)-protein, FMDH(formate dehydrogenase)-protein or DHAS(dihydroxyacetonesynthase)-protein of a methylotrophic organism.

15. The method according to any of claims 1 to 11, characterised in that the DNA sequence coding for the protein to be expressed is obtained from yeast, preferably Hansenula polymorpha.

16. The method according to any of claims 1 to 15, characterised in that the expression cassette is carried by plasmid pH19 of figure 2, said plasmid being obtainable by combining pHARS1, digested with EcoR1 and BamHI, with a 4.0 kb EcoRV-BamHI fragment from the Hansenula polymorpha genome carrying the MOX gene of Hansenula polymorpha in a manner known per se.

17. The method according to any of claims 1 to 16, characterised in that the DNA sequence to be expressed is coding for any one of the proteins of the following group:
Hepatitis B antigens, any other viral antigen, protease inhibitors especially trypsin inhibitors, growth factors, colony stimulating factors, blood anticoagulation factors, factors regulating blood coagulation and clot dissolving factors, bacterial toxins and antigens, antigens from parasites, especially from Plasmodium, enzymes for the food industry.

18. A process for the production of a protein in a methylotrophic organism characterised in that any of the mutants produced according to claims 1 to 17 is cultivated and protein synthesis is induced.

19. A process according to claim 18 characterised in that protein synthesis is induced by depletion of repressing carbon sources, and/or optionally by addition of methanol.

20. A process according to claims 18 or 19, characterised in that protein synthesis results in over expression of the protein encoded on the expression cassette.

21. A process according to any of claims 18 to 20, characterised in that the protein is methanol oxidase.

22. A method for the selection of methanol oxidase deficient mutants of methyltrophic yeast wherein a catalase deficient yeast is grown by using a medium containing methanol and a carbon source in an amount which does not cause repression of methanol inducible genes, and wherein the clones capable to grow on that medium are isolated as methanol oxidase deficient yeasts.

23. A method according to claim 22, characterised in that the catalase deficient yeast is a yeast of one of the genera Pichia, Candida or Hansenula.

24. A method according to claim 22, characterised in that the catalase deficient yeast is Hansenula polymorpha MCT-75(DSM 4890).

25. A method for the preparation of a mutant strain of a methylotrophic yeast containing at least one copy of an expression cassette, the original chromosome of said methylotrophic yeast being defective in effecting synthesis of active methanol oxidase and active catalase, wherein said mutant strain of a methylotrophic yeast is produced by transforming said methylotrophic yeast with a plasmid carrying the expressing cassette and the plasmid is integrated.

26. A method according to claim 25, characterised in that the plasmid is an autonomously replicating plasmid, preferably an autonomously replicating of high copy number.

27. A method according to claim 26, characterised in that the organism after transformation is grown under non-selective conditions for several generations and subsequently mitotically stable transformants containing at least one copy of the expression cassette are selected by growth under selective conditions.

28. A method according to any of claims 25 to 27, characterised in that the methylotrophic yeast is Hansenula polymorpha 1N or Hansenula polymorpha 3N.

29. A method for obtaining Hansenula polymorpha 1N characterised in that Hansenula polymorpha 1N (DSM4888) is cultivated and recovered.

30. A method for obtaining Hansenula polymorpha 3N characterised in that Hansenula polymorpha 3N (DSM4889) is cultivated and recovered.

## Patentansprüche (Patentansprüche für folgende(n) Vertragsstaat(en): AT, BE, CH, LI, DE, FR, GB, GR, IT, LU, NL, SE)

1. Mutantenstamm einer methylotrophen Hefe, in dem das ursprüngliche Chromosom der methylotrophen Hefe hinsichtlich der Bewirkung der Synthese von aktiver Methanoloxidase und aktiver Katalase defekt ist, wobei die Mutante mindestens eine Expressionskassette erhalten hat, die ein 5'-Transkriptionsregulon eines Methanol-induzierbaren Genes, eine DNA-Sequenz, die für ein zu exprimierendes Protein kodiert, und ggf. einen Terminator umfaßt, wobei die Expression der DNA-Sequenz unter Kontrolle des Regulons steht.

2. Mutante nach Anspruch 1, dadurch gekennzeichnet, daß die methylotrophe Hefe einer der Gattungen Pichia, Candida oder Hansenula, bevorzugt der Art Hansenula polymorpha, Pichia pastoris, Pichia methylotropica oder Candida boidinii angehört.

3. Mutante nach einem der Ansprüche 1 oder 2, dadurch gekennzeichnet, daß die Expressionskassette auf einem rekombinanten Plasmid zur Verfügung gestellt wird, das ein oder mehrere selektive Markergene und ein oder mehrere Replikons umfaßt, wobei mindestens eines dieser Replikons eine autonome Replikation in der methylotrophen Hefe bewirken kann.

4. Mutante nach Anspruch 3, dadurch gekennzeichnet, daß das zur Verfügung gestellte Replikon HARS 1 aus Hansenula oder eine funktionell äquivalente Sequenz ist.

5. Mutante nach Anspruch 3 oder 4, dadurch gekennzeichnet, daß mindestens eines der auf dem Plasmid bereitgestellten Replikons von einem prokaryontischen Wirtsorganismus erkannt wird.

6. Mutante nach einem der Ansprüche 3 bis 5, dadurch gekennzeichnet, daß das Plasmid in einer Kopienzahl von ungefähr 10 bis ungefähr 300 Kopien, bevorzugt 30 bis 100 Kopien, vorhanden ist.

7. Mutante nach Anspruch 1 oder 2, dadurch gekennzeichnet, daß mindestens eine Expressionskassette an mindestens einer Stelle des Chromosomes zur Verfügung gestellt wird.

8. Mutante nach Anspruch 7, dadurch gekennzeichnet, daß sie durch Integration mindestens eines Plasmides, das die Expressionskassette trägt, in das Chromosom einer Hefe der Gattung Hansenula erhältlich ist.

9. Mutante nach Anspruch 7 oder 8, dadurch gekennzeichnet, daß die Anzahl der in das Genom der methylotrophen Hefe eingeführten Expressionskassetten 1 bis ungefähr 300, bevorzugt 2 bis 300 oder 2 bis 150 beträgt.

10. Mutante nach einem der Ansprüche 1 bis 9, dadurch gekennzeichnet, daß das Regulon aus dem MOX-Gen, bevorzugt dem MOX-Gen von Hansenula polymorpha, abgeleitet ist.

11. Mutante nach einem der Ansprüche 1 bis 10, dadurch gekennzeichnet, daß die DNA-Sequenz für ein Protein mit der biologischen Aktivität des MOX(Methanoloxidase)-Proteines, FMDH(Formiatdehydrogenase)-Proteines oder DHAS(Dihydroxyacetonsynthase)-Proteines eines methylotrophen Organismus kodiert.

12. Mutante nach einem der Ansprüche 1 bis 11, dadurch gekennzeichnet, daß die für das zu exprimierende Protein kodierende DNA-Sequenz aus Hefe, bevorzugt Hansenula polymorpha, erhalten ist.

13. Mutante nach einem der Ansprüche 1 bis 12, dadurch gekennzeichnet, daß die Expressionskassette von dem Plasmid pH19 der Figur 2 getragen wird, wobei das Plasmid durch Kombination von pHARS1, abgebaut mit EcoRI und BamHI, mit einem EcoRV-BamHI-Fragment von 4,0 kb aus dem Hansenula polymorpha-Genom, das das MOX-Gen von Hansenula polymorpha trägt, auf an sich bekannte Weise erhältlich ist.

14. Mutante nach einem der Ansprüche 1 bis 13, dadurch gekennzeichnet, daß die zu exprimierende DNA-Sequenz für eines der Proteine der folgenden Gruppe kodiert:
Hepatitis-B-Antigene, jedes andere virale Antigen, Protease-Inhibitoren, insbesondere Trypsin-Inhibitoren, Wachstumsfaktoren, Kolonie-stimulierende Faktoren, Antikoagulationsfaktoren aus dem Blut, Faktoren, die die Blutgerinnung regulieren, und Blutgerinnsel-auflösende Faktoren, bakterielle Toxine und Antigene, Antigene aus Parasiten, insbesondere von Plasmodium, Enzyme für die Nahrungsmittelindustrie.

15. Verfahren zum Erzeugen eines Proteines in einem methylotrophen Organismus, dadurch gekennzeichnet, daß eine der Mutanten der Ansprüche 1 bis 14 kultiviert und Proteinsynthese induziert wird.

16. Verfahren nach Anspruch 15, dadurch gekennzeichnet, daß die Proteinsynthese durch Verringerung reprimierender Kohlenstoffquellen und/oder ggf. durch Zufügen von Methanol induziert wird.

17. Verfahren nach einem der Ansprüche 15 oder 16, dadurch gekennzeichnet, daß die Proteinsynthese zu einer Überexpression des auf der Expressionskassette kodierten Proteines führt.

18. Verfahren nach einem der Ansprüche 15 bis 17, dadurch gekennzeichnet, daß das Protein Methanoloxidase ist.

19. Verfahren zur Selektion von Methanoloxidase-defizienten Mutanten einer methylotrophen Hefe, wobei eine Katalasedefiziente Hefe unter Verwendung eines Mediums, das Methanol und eine Kohlenstoffquelle in einer Menge enthält, die keine Repression von Methanol-induzierbaren Genen verursacht, wachsengelassen wird, und wobei Klone, die auf diesem Medium wachsen können, als Methanoloxidase-defiziente Hefen isoliert werden.

20. Verfahren nach Anspruch 19, dadurch gekennzeichnet, daß die Katalase-defiziente Hefe eine Hefe einer der Gattungen Pichia, Candida oder Hansenula ist.

21. Verfahren nach Anspruch 19, dadurch gekennzeichnet, daß die Katalase-defiziente Hefe Hansenula polymorpha MCT-75(DSM 4890) ist.

22. Verfahren zum Herstellen eines mindestens eine Kopie einer Expressionskassette enthaltenden Mutantenstammes einer methylotrophen Hefe, wobei das ursprüngliche Chromosom der methylotrophen Hefe hinsichtlich der Bewirkung der Synthese von aktiver Methanoloxidase und aktiver Katalase defekt ist, wobei der Mutantenstamm einer methylotrophen Hefe durch Transformieren der methylotrophen Hefe mit einem Plasmid, das die Expressionskassette trägt, ggf. gefolgt von der Integration des Plasmides, erzeugt wird.

23. Verfahren nach Anspruch 22, dadurch gekennzeichnet, daß das Plasmid ein autonom replizierendes Plasmid, bevorzugt ein autonom replizierendes Plasmid mit hoher Kopienzahl ist.

24. Verfahren nach Anspruch 23, dadurch gekennzeichnet, daß der Organismus nach der Transformation mehrere Generationen lang unter nicht-selektiven Bedingungen wachsengelassen wird und anschließend mitotisch stabile Transformanten, die mindestens eine Kopie der Expressionskassette enthalten, durch Wachstum unter selektiven Bedingungen selektioniert werden.

25. Verfahren nach einem der Ansprüche 22 bis 24, dadurch gekennzeichnet, daß die methylotrophe Hefe Hansenula polymorpha 1N oder Hansenula polymorpha 3N ist.

26. Hansenula polymorpha 1N (DSM 4888).

27. Hansenula polymorpha 3N (DSM 4889).

## Patentansprüche (Patentansprüche für folgende(n) Vertragsstaat(en): ES)

1. Verfahren zum Herstellen eines mindestens eine Kopie einer Expressionskassette enthaltenden Mutantenstammes einer methylotrophen Hefe, wobei das ursprüngliche Chromosom der methylotrophen Hefe hinsichtlich der Bewirkung der Synthese von aktiver Methanoloxidase und aktiver Katalase defekt ist, und wobei der Mutantenstamm der methylotrophen Hefe durch Transformieren der methylotrophen Hefe mit einem Plasmid, das die Expressionskassette trägt, erzeugt wird und der Transformation ggf. die Integration des Plasmides folgt, wobei die Expressionskassette ein 5'-Transkriptionsregulon eines Methanol-induzierbaren Genes, eine für ein zu exprimierendes Protein kodierende DNA-Sequenz und gff. einen Terminator umfaßt und wobei die Expression der DNA-Sequenz unter Kontrolle des Regulons steht.

2. Verfahren nach Anspruch 1, dadurch gekennzeichnet, daß das Plasmid ein autonom replizierendes Plasmid, bevorzugt ein autonom replizierendes Plasmid mit hoher Kopienzahl ist.

3. Verfahren nach Anspruch 2, dadurch gekennzeichnet, daß der Organismus nach Transformation mehrere Generationen lang unter nicht-selektiven Bedingungen wachsengelassen wird und anschließend mitotisch stabile Transformanten, die mindestens eine Kopie der Expressionskassette enthalten, durch Wachstum unter selektiven Bedingungen selektioniert werden.

4. Verfahren nach einem der Ansprüche 1 bis 3, dadurch gekennzeichnet, daß die methylotrophe Hefe einer der Gattungen Pichia, Candida oder Hansenula, bevorzugt einer der Arten Hansenula polymorpha, Pichia pastoris, Pichia methylotropica oder Candida boidinii angehört.

5. Verfahren nach Anspruch 4, dadurch gekennzeichnet, daß die methylotrophe Hefe Hansenula polymorpha 1N oder Hansenula polymorpha 3N ist.

6. Verfahren nach einem der Ansprüche 1 bis 5, dadurch gekennzeichnet, daß die Expressionskassette auf einem rekombinanten Plasmid zur Verfügung gestellt wird, das ein oder mehrere selektive Markergene und ein oder mehrere Replikons enthält, wobei mindestens eines dieser Replikons eine autonome Replikation in der methylotrophen Hefe bewirken kann.

7. Verfahren nach Anspruch 6, dadurch gekennzeichnet, daß das zur Verfügung gestellte Replikon HARS1 aus Hansenula oder eine funktionell äquivalente Sequenz ist.

8. Verfahren nach einem der Ansprüche 1 bis 7, dadurch gekennzeichnet, daß mindestens eines der auf dem Plasmid bereitgestellten Replikons von einem prokaryontischen Wirtsorganismus erkannt wird.

9. Verfahren nach einem der Ansprüche 6 bis 8, dadurch gekennzeichnet, daß das Plasmid in einer Kopienzahl von ungefähr 10 bis ungefähr 300 Kopien, bevorzugt 30 bis 100 Kopien vorhanden ist.

10. Verfahren nach einem der Ansprüche 1 bis 5, dadurch gekennzeichnet, daß mindestens eine Expressionskassette an mindestens einer Stelle des Chromosomes zur Verfügung gestellt wird.

11. Verfahren nach Anspruch 10, dadurch gekennzeichnet, daß mindestens ein die Expressionskassette tragendes Plasmid in das Chromosom einer Hefe der Gattung Hansenula integriert wird.

12. Verfahren nach Anspruch 11, dadurch gekennzeichnet, daß die Anzahl der in das Genom der methylotrophen Hefe eingeführten Expressionskassetten 1 bis ungefähr 300, bevorzugt 2 bis 300 oder 2 bis 150 beträgt.

13. Verfahren nach einem der Ansprüche 1 bis 12, dadurch gekennzeichnet, daß das Regulon aus dem MOX-Gen, bevorzugt dem MOX-Gen von Hansenula polymorpha, abgeleitet ist.

14. Verfahren nach einem der Ansprüche 1 bis 13, dadurch gekennzeichnet, daß die DNA-Sequenz für ein Protein mit der biologischen Aktivität des MOX(Methanoloxidase)-Proteines, FMDH(Formiatdehydrogenase)-Proteines oder DHAS(Dihydroxyacetonsynthase)-Proteines eines methylotrophen Organismus kodiert.

15. Verfahren nach einem der Ansprüche 1 bis 11, dadurch gekennzeichnet, daß die für das zu exprimierende Protein kodierende DNA-Sequenz aus Hefe, bevorzugt Hansenula polymorpha, erhalten ist.

16. Verfahren nach einem der Ansprüche 1 bis 15, dadurch gekennzeichnet, daß die Expressionskassette von dem Plasmid pH19 der Figur 2 getragen wird, wobei das Plasmid durch Kombination von pHARS1, abgebaut mit EcoRI und BamHI, mit einem EcoRV-BamHI-Fragment von 4,0 kb aus dem Hansenula polymorpha-Genom, das das MOX-Gen von Hansenula polymorpha trägt, auf an sich bekannte Weise kombiniert wird.

17. Verfahren nach einem der Ansprüche 1 bis 16, dadurch gekennzeichnet, daß die zu exprimierende DNA-Sequenz für eines der Proteine der folgenden Gruppe kodiert:
Hepatitis-B-Antigene, jedes andere virale Antigen, Protease-Inhibitoren, insbesondere Trypsin-Inhibitoren, Wachstumsfaktoren, Kolonie-stimulierende Faktoren, Antikoagulationsfaktoren aus dem Blut, Faktoren, die die Blutgerinnung regulieren, und Blutgerinnsel-auflösende Faktoren, bakterielle Toxine und Antigene, Antigene aus Parasiten, insbesondere aus Plasmodium, Enzyme für die Nahrungsmittelindustrie.

18. Verfahren zum Erzeugen eines Proteines in einem methylotrophen Organismus, dadurch gekennzeichnet, daß eine der nach den Ansprüchen 1 bis 17 erzeugten Mutanten kultiviert und die Proteinsynthese induziert wird.

19. Verfahren nach Anspruch 18, dadurch gekennzeichnet, daß die Proteinsynthese durch Verringerung reprimierender Kohlenstoffquellen und/oder ggf. durch Zufügen von Methanol induziert wird.

20. Verfahren nach den Ansprüchen 18 oder 19, dadurch gekennzeichnet, daß die Proteinsynthese zu einer Überexpression des auf der Expressionskassette kodierten Proteines führt.

21. Verfahren nach einem der Ansprüche 18 bis 20, dadurch gekennzeichnet, daß das Protein Methanoloxidase ist.

22. Verfahren zur Selektion von Methanoloxidase-defizienten Mutanten einer methylotrophen Hefe, worin eine Katalase-defiziente Hefe unter Verwendung eines Mediums, das Methanol und eine Kohlenstoffquelle in einer Menge enthält, die keine Repression von Methanol-induzierbaren Genen bewirkt, wachsengelassen wird, und wobei Klone, die auf diesem Medium wachsen können, als Methanoloxidase-defiziente Hefen isoliert werden.

23. Verfahren nach Anspruch 22, dadurch gekennzeichnet, daß die Katalase-defiziente Hefe eine Hefe einer der Gattungen Pichia, Candida oder Hansenula ist.

24. Verfahren nach Anspruch 22, dadurch gekennzeichnet, daß die Katalase-defiziente Hefe Hansenula polymorpha MCT-75(DSM 4890) ist.

25. Verfahren zum Herstellen eines mindestens eine Kopie einer Expressionskassette enthaltenden Mutantenstammes einer methylotrophen Hefe, wobei das ursprüngliche Chromosom der methylotrophen Hefe hinsichtlich der Bewirkung der Synthese von aktiver Methanoloxidase und aktiver Katalase defekt ist, wobei der Mutantenstamm einer methylotrophen Hefe durch Transformieren der methylotrophen Hefe mit einem die Expressionskassette tragenden Plasmid erzeugt wird und das Plasmid integriert wird.

26. Verfahren nach Anspruch 25, dadurch gekennzeichnet, daß das Plasmid ein autonom replizierendes Plasmid , bevorzugt ein autonom replizierendes Plasmid mit hoher Kopienzahl ist.

27. Verfahren nach Anspruch 26, dadurch gekennzeichnet, daß der Organismus nach der Transformation mehrere Generationen lang unter nicht-selektiven Bedingungen wachsengelassen wird und anschließend mitotisch stabile Transformanten, die mindestens eine Kopie der Expressionskassette enthalten, durch Wachstum unter selektiven Bedingungen selektioniert werden.

28. Verfahren nach einem der Ansprüche 25 bis 27, dadurch gekennzeichnet, daß die methylotrophe Hefe Hansenula polymorpha 1N oder Hansenula polymorpha 3N ist.

29. Verfahren zum Erhalten von Hansenula polymorpha 1N, dadurch gekennzeichnet, daß Hansenula polymorpha 1N (DSM 4888) kultiviert und gewonnen wird.

30. Verfahren zum Erhalten von Hansenula polymorpha 3N, dadurch gekennzeichnet, daß Hansenula polymorpha 3N (DSM 4889) kultiviert und gewonnen wird.

## Revendications (Revendications pour l'(les) Etat(s) contractant(s) suivant(s): AT, BE, CH, LI, DE, FR, GB, GR, IT, LU, NL, SE)

1. Souche mutante d'une levure méthylotrophe, dans laquelle le chromosome d'origine de cette levure méthylotrophe est défectif eu égard à la synthèse de méthanoloxydase active et de catalase active, ayant acquis au moins une cassette d'expression comprenant un régulon de transcription à partir de l'extrémité 5' d'un gène inductible par le méthanol, une séquence d'ADN codant pour une protéine destinée à être exprimée, et éventuellement un terminateur, l'expression de ladite séquence d'ADN étant contrôlée par le régulon.

2. Mutant selon la revendication 1, caractérisé en ce que la levure méthylotrophe est une levure choisie parmi les genres Pichia, Candida ou Hansenula, correspondant de préférence aux espèces Hansenula polymorpha, Pichia pastoris, Pichia methylotropica ou Candida boidinii.

3. Mutant selon la revendication 1 ou 2, caractérisé en ce que la cassette d'expression est incorporée dans un plasmide recombinant comprenant un ou plusieurs gènes de marquage sélectifs, et un ou plusieurs réplicons, au moins un de ces réplicons étant capable d'effectuer une réplication autonome dans ladite levure méthylotrophe.

4. Mutant selon la revendication 3, caractérisé en ce que le réplicon fourni est HARS 1 dérivé de Hansenula, ou une séquence fonctionnellement équivalente.

5. Mutant selon la revendication 3 ou 4, caractérisé en ce qu'au moins l'un des réplicons présents dans le plasmide, est reconnu par un organisme-hôte procaryote.

6. Mutant selon l'une quelconque des revendications 3 à 5, caractérisé en ce que le plasmide est présent selon un nombre de copies d'environ 10 à environ 300 copies, de préférence de 30 à 100 copies.

7. Mutant selon la revendication 1 ou 2, caractérisé en ce que au moins une cassette d'expression est prévue dans au moins un site du chromosome.

8. Mutant selon la revendication 7, caractérisé en ce qu'il peut être obtenu par intégration d'au moins un plasmide comportant la cassette d'expression, dans le chromosome d'une levure du genre Hansenula.

9. Mutant selon la revendication 7 ou 8, caractérisé en ce que le nombre de cassettes d'expression, introduites dans le génome de la levure méthylotrophe, est de 1 à environ 300, de préférence de 2 à 300, ou de 2 à 150.

10. Mutant selon l'une quelconque des revendications 1 à 9, caractérisé en ce que le régulon est dérivé du gêne MOX, de préférence le gène MOX de Hansenula polymorpha.

11. Mutant selon l'une quelconque des revendications 1 à 10, caractérisé en ce que la séquence d'ADN code pour une protéine ayant l'activité biologique de la protéine MOX (méthanol oxydase), de la protéine FMDH (formate déshydrogénase) ou de la protéine DHAS (dihydroxyacétone-synthase) d'un organisme méthylotrophe.

12. Mutant selon l'une quelconque des revendications 1 à 11, caractérisé en ce que la séquence d'ADN codant pour la protéine destinée à être exprimée, est obtenue à partir d'une levure, de préférence Hansenula polymorpha.

13. Mutant selon l'une quelconque des revendications 1 à 12, caractérisé en ce que la cassette d'expression est portée par le plasmide pH19 de la figure 2, ce plasmide pouvant être obtenu en combinant pHARS1 digéré avec EcoRI et BamHI, avec un fragment EcoRV-BamHI de 4,0 kb, dérivé du génome de Hansenula polymorpha, comportant le gène MOX de Hansenula polymorpha, de façon connue en soi.

14. Mutant selon l'une quelconque des revendications 1 à 13, caractérisé en ce que la séquence d'ADN destinée à être exprimée, code pour l'une quelconque des protéines du groupe suivant :
les antigènes de l'hépatite B, n'importe quel autre antigène viral, les inhibiteurs de protéase, en particulier les inhibiteurs de trypsine, les facteurs de croissance, les facteurs de stimulation de colonie, les facteurs anticoagulants sanguins, les facteurs de régulation de coagulation sanguine et les facteurs de dissolution de caillot, les toxines et les antigènes bactériens, les antigènes de parasites, en particulier de Plasmodium, les enzymes pour l'industrie alimentaire.

15. Procédé de production d'une protéine dans un organisme méthylotrophe, caractérisé en ce que l'on cultive l'un quelconque des mutants des revendications 1 à 14 et on induit la synthèse de protéine.

16. Procédé selon la revendication 15, caractérisé en ce que la synthèse de protéine est induite par épuisement de sources de carbone répressives, et/ou éventuellement par addition de méthanol.

17. Procédé selon la revendication 15 ou 16, caractérisé en ce que la synthèse de protéine résulte en l'expression excessive de la protéine codée dans la cassette d'expression.

18. Procédé selon l'une quelconque des revendications 15 à 17, caractérisé en ce que la protéine est la méthanoloxydase.

19. Procédé de sélection de mutants déficients en méthanoloxydase de levure méthylotrophe, selon lequel on cultive une levure déficiente en catalase, en employant un milieu contenant du méthanol et une source de carbone, selon une quantité qui ne provoque pas la répression des gènes inductibles par le méthanol, et selon lequel les clones capables de se développer sur ce milieu sont isolés en tant que levures déficientes en méthanoloxydase.

20. Procédé selon la revendication 19, caractérisé en ce que la levure déficiente en catalase est une levure appartenant à l'un des genres choisis parmi Pichia, Candida ou Hansenula.

21. Procédé selon la revendication 19, caractérisé en ce que la levure déficiente en catalase est Hansenula polymorpha MCT-75 (DSM 4890).

22. Procédé de préparation d'une souche mutante d'une levure méthylotrophe contenant au moins une copie d'une cassette d'expression, le chromosome d'origine de cette levure méthylotrophe étant défectif à l'égard de la synthèse de méthanoloxydase active et de catalase active, selon lequel on produit la souche mutante d'une levure méthylotrophe, en transformant cette levure méthylotrophe avec un plasmide comportant la cassette d'expression, et on intègre ensuite éventuellement le plasmide.

23. Procédé selon la revendication 22, caractérisé en ce que le plasmide est un plasmide auto-réplicable, de préférence un plasmide auto-réplicable en un nombre de copies élevé.

24. Procédé selon la revendication 23, caractérisé en ce que l'organisme est cultivé, après transformation, dans des conditions non sélectives, pendant plusieurs générations, et on sélectionne ensuite des transformants stables à l'égard de la mitose, contenant au moins une copie de la cassette d'expression, par culture dans des conditions sélectives.

25. Procédé selon l'une quelconque des revendications 22 à 24, caractérisé en ce que la levure méthylotrophe est Hansenula polymorpha 1N ou Hansenula polymorpha 3N.

26. Hansenula polymorpha 1N (DSM 4888).

27. Hansenula polymorpha 3N (DSM 4889).

## Revendications (Revendications pour l'(les) Etat(s) contractant(s) suivant(s): ES)

1. Procédé de préparation d'une souche mutante d'une levure méthylotrophe, contenant au moins une copie d'une cassette d'expression, selon lequel le chromosome d'origine de cette levure méthylotrophe est défectif eu égard à la synthèse de méthanoloxydase active et de catalase active, et selon lequel la souche mutante d'une levure méthylotrophe est produite en transformant cette levure méthylotrophe à l'aide d'un plasmide comportant la cassette d'expression, cette transformation étant éventuellement suivie par l'intégration du plasmide, la cassette d'expression comprenant un régulon de transcription à partir de l'extrémité 5' d'un gène inductible par le méthanol, une séquence d'ADN codant pour une protéine destinée à être exprimée, et éventuellement un terminateur, et l'expression de ladite séquence d'ADN étant contrôlée par le régulon.

2. Procédé selon la revendication 1, caractérisé en ce que le plasmide est un plasmide auto-réplicable, de préférence un plasmide auto-réplicable à nombre de copies élevé.

3. Procédé selon la revendication 2, caractérisé en ce que l'organisme, après transformation, est cultivé dans des conditions non sélectives pendant plusieurs générations, puis on sélectionne des transformants stables à l'égard de la mitose contenant au moins une copie de la cassette d'expression, par culture dans des conditions sélectives.

4. Procédé selon l'une quelconque des revendications 1 à 3, caractérisé en ce que la levure méthylotrophe, est une levure choisie parmi les genres Pichia, Candida ou Hansenula, correspondant de préférence aux espèces Hansenula polymorpha, Pichia pastoris, Pichia methylotropica ou Candida boidinii.

5. Procédé selon la revendication 4, caractérisé en ce que la levure méthylotrophe est Hansenula polymorpha 1N ou Hansenula polymorpha 3N.

6. Procédé selon l'une quelconque des revendications 1 à 5, caractérisé en ce que la cassette d'expression est incorporée dans un plasmide recombinant comprenant un ou plusieurs gènes de marquage sélectif, et un ou plusieurs réplicons, au moins un de ces réplicons étant capable d'effectuer une réplication autonome dans ladite levure méthylotrophe.

7. Procédé selon la revendication 6, caractérisé en ce que le réplicon fourni est HARS 1 dérivé de Hansenula, ou une séquence fonctionnellement équivalente.

8. Procédé selon les revendications 1 à 7, caractérisé en ce qu'au moins l'un des réplicons présents dans le plasmide, est reconnu par un organisme-hôte procaryote.

9. Procédé selon l'une quelconque des revendications 6 à 8, caractérisé en ce que le plasmide est présent selon un nombre de copies d'environ 10 à environ 300 copies, de préférence de 30 à 100 copies.

10. Procédé selon l'une des revendications 1 à 5, caractérisé en ce que la ou chaque cassette d'expression est prévue dans au moins un site du chromosome.

11. Procédé selon la revendication 10, caractérisé en ce qu'au moins un plasmide comportant la cassette d'expression est intégrée dans le chromosome d'une levure du genre Hansenula.

12. Procédé selon la revendication 11, caractérisé en ce que le nombre de cassettes d'expression, introduites dans le génome de la levure méthylotrophe, est de 1 à environ 300, de préférence de 2 à 300, ou de 2 à 150.

13. Procédé selon l'une quelconque des revendications 1 à 12, caractérisé en ce que le régulon est dérivé du gène MOX, de préférence le gène MOX de Hansenula polymorpha.

14. Procédé selon l'une quelconque des revendications 1 à 13, caractérisé en ce que la séquence d'ADN code pour une protéine ayant l'activité biologique de la protéine MOX (méthanoloxydase), de la protéine FMDH (formate déshydrogénase) ou de la protéine DHAS (dihydroxyacétone-synthase) d'un organisme méthylotrophe.

15. Procédé selon l'une quelconque des revendications 1 à 11, caractérisé en ce que la séquence d'ADN codant pour la protéine destinée à être exprimée, est obtenue à partir d'une levure, de préférence Hansenula polymorpha.

16. Procédé selon l'une quelconque des revendications 1 à 15, caractérisé en ce que la cassette d'expression est portée par le plasmide pH19 de la figure 2, ce plasmide pouvant être obtenu en combinant pHARS1 digéré avec EcoRI et BamHI, avec un fragment EcoRV-BamHI de 4,0. kb, dérivé du génome de Hansenula polymorpha, comportant le gène MOX de Hansenula polymorpha, de façon connue en soi.

17. Procédé selon l'une quelconque des revendications 1 à 16, caractérisé en ce que la séquence d'ADN destinée à être exprimée, code pour l'une quelconque des protéines du groupe suivant :
les antigènes de l'hépatite B, n'importe quel autre antigène viral, les inhibiteurs de protéase, en particulier les inhibiteurs de trypsine, les facteurs de croissance, les facteurs de stimulation de colonie, les facteurs anticoagulants sanguins, les facteurs de régulation de coagulation sanguine et les facteurs de dissolution de caillot, les toxines et les antigènes bactériens, les antigènes de parasites, en particulier de Plasmodium, les enzymes pour l'industrie alimentaire.

18. Procédé de production d'une protéine dans un organisme méthylotrophe, caractérisé en ce que l'on cultive l'un quelconque des mutants des revendications 1 à 17 et on induit la synthèse de protéine.

19. Procédé selon la revendication 18, caractérisé en ce que la synthèse de protéine est induite par épuisement de sources de carbone répressives, et/ou éventuellement par addition de méthanol.

20. Procédé selon la revendication 18 ou 19, caractérisé en ce que la synthèse de protéine résulte en l'expression excessive de la protéine codée dans la cassette d'expression.

21. Procédé selon l'une quelconque des revendications 18 à 20, caractérisé en ce que la protéine est la méthanoloxydase.

22. Procédé de sélection de mutants déficients en méthanol oxydase de levure méthylotrophe, selon lequel on cultive une levure déficiente en catalase, en employant un milieu contenant du méthanol et une source de carbone, selon une quantité qui ne provoque pas la répression des gènes inductibles par le méthanol, et selon lequel les clones capables de se développer sur ce milieu sont isolés en tant que levures déficientes en méthanoloxydase.

23. Procédé selon la revendication 22, caractérisé en ce que la levure déficiente en catalase est une levure appartenant à l'un des genres choisis parmi Pichia, Candida ou Hansenula.

24. Procédé selon la revendication 22, caractérisé en ce que la levure déficiente en catalase est Hansenula polymorpha MCT-75 (DSM 4890).

25. Procédé de préparation d'une souche mutante d'une levure méthylotrophe contenant au moins une copie d'une cassette d'expression, le chromosome d' origine de cette levure méthylotrophe étant défectif à l'égard de la synthèse de méthanoloxydase active et de catalase active, selon lequel on produit la souche mutante d'une levure méthylotrophe, en transformant cette levure méthylotrophe avec un plasmide comportant la cassette d'expression, et on intègre ensuite le plasmide.

26. Procédé selon la revendication 25, caractérisé en ce que le plasmide est un plasmide auto-réplicable, de préférence un plasmide auto-réplicable selon un nombre de copies élevé.

27. Procédé selon la revendication 26, caractérisé en ce que l'organisme est cultivé, après transformation, dans des conditions non sélectives, pendant plusieurs générations, et on sélectionne ensuite des transformants stables à l'égard de la mitose, contenant au moins une copie de la cassette d'expression, par culture dans des conditions sélectives.

28. Procédé selon l'une quelconque des revendications 25 à 27, caractérisé en ce que la levure méthylotrophe, est Hansenula polymorpha 1N ou Hansenula polymorpha 3N.

29. Procédé d'obtention d'Hansenula polymorpha 1N, caractérisé en ce que l'on cultive et on récupère Hansenula polymorpha 1N (DSM 4888).

30. Procédé d'obtention d'Hansenula polymorpha 3N, caractérisé en ce que l'on cultive et on récupère Hansenula polymorpha 3N (DSM 4889).
